# EUROPÄISCHE PATENTSCHRIFT

(11) **EP 2 671 610 B1**
(45) Veröffentlichungstag und Bekanntmachung des Hinweises auf die Patenterteilung: **16.09.2015**
(21) Anmeldenummer: 13002606.5
(22) Anmeldetag: 17.05.2013
(51) Int. Cl.: A61M 37/00

(54) **Nadelmodul mit automatischer Frequenz- und Amplitudenanpassung**
Nail module with automatic frequency and amplitude matching
Module d'aiguille avec adaptation automatique de la fréquence et de l'amplitude

(30) Priorität: 22.05.2012 DE 102012009991
(43) Veröffentlichungstag der Anmeldung: 11.12.2013
(73) Patentinhaber: Blank, Anton, 70173 Stuttgart (DE)
(72) Erfinder: Blank, Anton, 70173 Stuttgart (DE)

(56) Entgegenhaltungen:
- EP-A1- 1 882 491
- DE-A1- 4 331 442
- US-A- 2 588 623

## Beschreibung

### Stand der Technik

Geräte zum Einbringen von Stoffen in die Haut werden zum Beispiel zum Einbringen von in Flüssigkeiten gelösten Farbpigmenten für Permanent Make-up (PMU) oder Tätowierungen genutzt und bestehen üblicherweise aus einer Antriebseinheit (Handgerät) zur Erzeugung einer repetierenden Hubbewegung mit einer ausgefahrenen und einer eingefahrenen Stellung, die auf eine Vorrichtung, die ein Stechelement, welches mit dem einzubringenden Stoff benetzt ist, enthält, um die Haut bei jeder Stechbewegung punktuell anzustechen, damit der Stoff in die Haut eindringen und in ihr verbleiben kann.

Eine Fülle von Patentdokumenten zeigen solche Vorrichtungen. Bei allen diesen bekannten Vorrichtungen ist es der Erfahrung des Anwenders überlassen, die optimale Frequenz und Amplitude der oszillierenden Nadel für die Behandlung der jeweiligen Hautregion einzustellen. Davon abgesehen, dass nur unter Nutzung der Patentanmeldungen DE102008031907, DE102011014196 und EP12001723 des gleichen Anmelders, die Amplitude überhaupt vom Anwender eingestellt werden kann, denn alle anderen Geräte lassen nur eine Veränderung der Frequenz zu, ist das alleinige Verändern der Stechfrequenz ein nur beschränkt wirksames Mittel, um die jeweilige Hautregion medizinisch und kosmetisch optimal behandeln zu können.

Nach dem Stand der Technik wird zudem bei allen bekannten Ausführungen die Nadel oder die Hülsen-Nadelkombination vom Antrieb direkt angetrieben, was aus hygienischen Gründen einer Trennung zwischen Antriebsraum und Arbeitsraum bedarf, um zu verhindern, dass Keime von einem zum anderen Behandelten über eine Kontaminierung des Antriebsraums erfolgen kann (Rücklaufschutz). Die Trennung von Antriebsraum zum Flüssigkeitsraum erfolgt nach dem Stand der Technik mittels elastischer Membranen oder nach DE102011014196 und EP12001723 vom gleichen Anmelder durch eine hochviskose Flüssigkeit.

Ein weiterer Nachteil der bekannten Vorrichtungen besteht in der Tatsache, dass sich die Nadelhülse, in der die Nadel oszilliert, durch abgelöste Hautreste zunehmend zusetzt und damit eine Unterbrechung der Behandlung zur Reinigung der zugesetzten Nadelhülse erforderlich wird, die vor der sichtbaren Zusetzung bereits zu Qualitätseinbußen bei den zuletzt behandelten Regionen geführt hatte, die nicht mehr rückgängig gemacht werden können.

### Die Erfindung

Aufgabe der Erfindung ist es, eine Vorrichtung zu schaffen, die ohne den Einsatz von Dichtungen hygienisch optimale Verhältnisse bereitstellt und die sich anhand der gewählten Nadel-Hüfsenkombination automatisch auf eine medizinisch und kosmetisch optimale Arbeitsamplitude und Arbeitsfrequenz für die zu behandelnde Hauregion einstellt und einem Zusetzen der Nadelhülse entgegen wirkt.

Diese Aufgabe wird erfindungsgemäß mit einer Vorrichtung nach dem Oberbegriff des unabhängigen Anspruchs 1 dadurch gelöst, dass die Nadel-Hülsenkombination 7 mit dem Gehäuse 1 fest gekoppelt ist, dadurch das gesamte Modul vom Antrieb in Schwingungen versetzt wird und ein Antrieb verwendet wird, der beispielsweise durch die im Nadelmodul veränderbare Masse 6 in seiner Frequenz und Amplitude beeinflusst werden kann und durch die hermetische Trennung von Antriebskammer 3 zur Flüssigkeitskammer 2 ein vollständiger Rücklaufschutz gewährleistet ist und die Nadelaussparung 8 dazu vorgesehen ist, einer Zusetzung der Nadel-Hülsenkombination entgegen zu wirken.

Ein wesentlicher Vorteil, welcher mit der Erfindung gegenüber dem Stand der Technik erreicht wird, besteht darin, dass eine Verwendung von elastischen Dichtungen oder hochviskosen Flüssigkeiten zur Abdichtung der beiden Kammern nicht mehr notwendig ist, um einen Rücklaufschutz zu gewährleisten, da die Nadel nicht mehr durch die Trennung von Flüssigkeitskammer 2 und Arbeitskammer 3 hindurch gehen muss, weil sie nicht mehr vom Antrieb 5 direkt angetrieben wird.

Ein weiterer wesentlicher Vorteil, welcher gegenüber dem Stand der Technik erreicht wird, besteht darin, dass die Lagerung der Nadel in einem Gleitlager, das Energie durch Reibung verbraucht, nicht mehr erforderlich ist, was zu einfacherer und preiswerterer Fertigung und kleineren Antrieben mit insgesamt höherem Wirkungsgrad führt.

Ein weiterer wesentlicher Vorteil der Erfindung gegenüber dem Stand der Technik besteht darin, dass das bisher notwendige Lagerspiel der Gleitlager für die Nadel, welches der Nadel eine für die Anwendung ungünstiges seitliches Spiel zulassen musste, um die Reibungsverluste des Lagers zu minimieren, ebenfalls vollständig entfällt, was wesentlich präzisere Ergebnisse in der Anwendung zulässt, da die Nadel keinerlei seitliches Spiel mehr aufweist und zudem zu einfacherer und preiswerterer Fertigung der Nadelmodule führt.

Eine weiterer wesentlicher Vorteil der Ausgestaltung der Erfindung wird erfindungsgemäß dadurch gebildet, mit der Zusatzmasse 6 unterschiedliche Nadelmodule mit unterschiedlichen Frequenzen und Amplituden hergestellt werden können, die exakt auf den jeweiligen Einsatzzweck abgestimmt werden können. Ein Wechsel der Nadel stellt damit die ideale Frequenz und ideale Amplitude automatisch ein.

Eine vorteilhafte Möglichkeit der Ausgestaltung der Erfindung ist, dass die Nadelaussparung 8 dafür sorgt, dass sich darin bildende Hautreste durch die bei der Anwendung stets durchgeführte Seitwärtsbewegung der Nadel von allein und stetig entfernen und daher dem Zusetzen der Nadel Hülsenkombination wirkungsvoll entgegen gewirkt wird.

Erfindungsgemäß werden die Hygieneanforderung zur Vermeidung von Arbeitsraum-Kontaminierung durch Keime auch ohne Dichtungen erfüllt.

Erfindungsgemäß werden an die jeweilige Anwendung optimal anpassbare Amplituden und Frequenzen alleine und automatisch durch das Nadelmodul eingestellt, so dass auch unerfahrene Anwender von Beginn an medizinisch und kosmetisch optimal arbeiten können.

Erfindungsgemäß wird ein Zusetzen der Nadel-Hülsenkombination durch Hautreste dadurch verhindert, dass die Kapillare am zur Haut zeigenden Ende ein Aussparung aufweist, die sich durch die während der Anwendung stets vorkommende seitliche Bewegungen ohne zusätzliches Zutun von Hautresten säubert.

Die Vorrichtung mit automatischer Frequenz- und Amplitudenanpassung entfaltet die beschriebenen Vorteile beim Anbringen von Permanent Make-Up und Tätowierungen in eine Haut.

### Bevorzugtes Ausführungsbeispiel der Erfindung

Die Erfindung wird nachfolgend anhand eines Ausführungsbeispiels unter Bezugnahme auf eine Zeichnung näher erläutert.

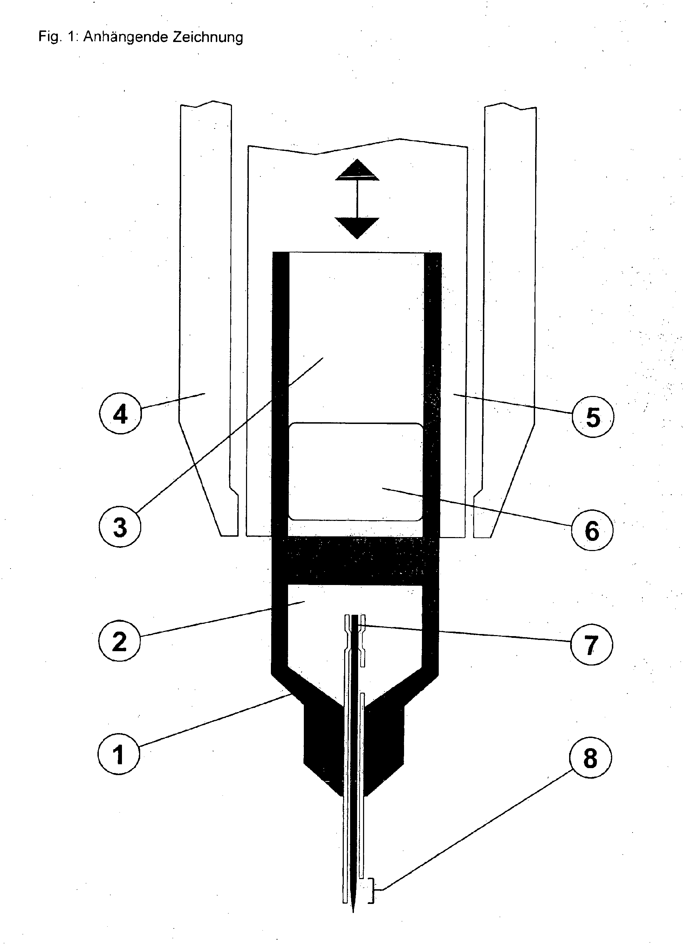

Fig. 1 zeigt eine Vorrichtung zur automatischen Frequenz- und Amplitudenanpassung durch ein erfindungsgemäßes Nadelmodul für das Anbringen von Permanent Make-Up und Tätowierungen in eine Haut, mit einem Gehäuse 1, das in eine vordere Flüssigkeitskammer 2 und eine hintere Antriebskammer 3, die zur Vermeidung von Kontaminationen der Arbeitskammer voneinander hermetisch getrennt sind, aufgeteilt ist. Das Gehäuse 1 koppelt zusammen mit der darin fest eingebauten Nadel-Hülsenkombination 7 lösbar mit dem Antrieb 5, der zur Beeinflussung seiner Amplitude und seiner Frequenz durch die zusätzliche Masse 6 ausgeführt ist, während die Nadelaussparung 8 ein Zusetzen der Nadel-Hülsenkombination durch Hautreste verhindert.

Die in der vorstehenden Beschreibung, der Zeichnung und den Ansprüchen offenbarten Merkmale der Erfindung können sowohl einzeln als auch in beliebiger Kombination für die Verwirklichung der Erfindung in ihren verschiedenen Ausführungsformen von Bedeutung sein. Der Schutzgegenstand wird durch die zugehörigen Ansprüche definiert.

## Patentansprüche

1. Vorrichtung zur automatischen Frequenz- und Amplitudenanpassung für ein Nadelmodul zum Anbringen von Permanent Make-Up und Tätowierungen in eine Haut, mit:
- einem Gehäuse 1
- einer im Gehäuse fest eingebauten Nadel-Hülsenkombination 7
- und einer Nadelaussparung 8
**dadurch gekennzeichnet, dass** mit Hilfe eines Antriebs 5 das Gehäuse 1, mit der darin fest montierten Nadel-Hülsenkombination 7, in eine repetierende Axialbewegung versetzt wird.

2. Vorrichtung nach Anspruch 1, dadurch gekenntzeichnet, dass das Gehäuse 1 eine vordere Flüssigkeitskammer 2 aufweist, die von einer hinteren Antriebskammer 3 hermetisch dicht getrennt ist.

3. Vorrichtung nach Anspruch 1, **dadurch gekennzeichnet, dass** das Gehäuse 1 als Einwegelement ausgeführt ist, das lösbar an einem Antrieb koppelbar ist.

4. Vorrichtung nach Anspruch 1, **dadurch gekennzeichnet, dass** durch ein Zusatzgewicht 6 oder eine entsprechend schwereres oder leichteres Gehäuse 1 eine Beeinflussung von Amplitude und Frequenz am Antrieb 5 durchführbar ist, die sich alleine durch Änderung der Gesamtmasse des Nadelmoduls so beeinflussen lässt, dass optimale medizinische und kosmetische Ergebnisse erzielbar sind.

5. Verwendung einer Vorrichtung nach einem der vorangegangenen Ansprüche zum Anbringen von Permanent Make-Up und Tätowierungen in eine Haut.

## Claims

1. An apparatus for automatic frequency- and amplitude matching for a needle-module for applying Permanent-Make-Up and Tattoos into a skin, comprising:
- a housing 1
- a needle-sleeve combination rigidly connected to the housing
- and a cut-out in the needle-sleeve 8
wherein the housing 1 with its built-in needle-sleeve combination 7 is driven in a repetitive axial movement by a drive 5.

2. The apparatus as in claim 1, wherein the housing 1 comprises a front liquid-chamber 2 which is separated and hermetically sealed from the rear drive-chamber 3.

3. The apparatus as in claim 1, wherein the housing 1 is carried out as a one-way element which is coupleable and detachable to a drive.

4. The apparatus as in claim 1, wherein an inducement of the amplitude and frequency of drive 5 can be performed by an additional weight 6 or an equivalent heavier or lighter housing 1, which solely can be affected by the variation of the total mass of the needle module so that optimal medical and cosmetic results can be achieved.

5. Use of an apparatus according to one of the preceding claims for applying Permanent Make-Up and Tattoos into a skin.

## Revendications

1. Dispositif d'adaptation de fréquence et d'amplitude automatique pour un outil à aiguille destiné à l'apport de permanent make-up ou de tatouage dans la peau
- doté d'un boîtier 1,
- d'une combinaison aiguille - manche 7 solidement encastrée dans le boîtier
- et d'une ouverture d'aiguille 8
**caractérisé par le fait que** le boîtier 1, ainsi que la combinaison aiguille - manche 7 solidement montée à l'intérieur, effectue un mouvement répété par rapport à un axe grâce à un moteur 5.

2. Dispositif conforme à l'exigence 1 **caractérisé par le fait que** le boîtier 1 comporte une chambre de rétention de liquide 2 située à l'avant qui est hermétiquement séparée d'une chambre d'entraînement 3 à l'arrière.

3. Dispositif conforme à l'exigence 1 **caractérisé par le fait que** le boîtier 1 forme un élément jetable, élément détachable que l'on accroche à un moteur.

4. Dispositif conforme à l'exigence 1 **caractérisé par le fait qu'**il permet d'adapter l'amplitude et la fréquence au moteur 5 par le biais d'un poids supplémentaire 6 ou d'un boîtier 1 plus lourd ou plus léger selon le cas, adaptation qui permet d'atteindre des résultats médicaux et cosmétiques optimaux rien qu'en modifiant le poids total de l'outil à aiguille.

5. Utilisation d'un dispositif conforme à l'une des exigences précédentes destiné à l'apport de permanent make-up et de tatouage dans la peau.
